(19) Europäisches Patentamt
European Patent Office
Office européen des brevets

(11) Publication number: **0 135 661**
**B1**

(12) # EUROPEAN PATENT SPECIFICATION

(45) Date of publication of patent specification: **07.10.87**

(51) Int. Cl.$^4$: **C 08 F 18/04,** C 08 F 2/26

(21) Application number: **84104759.0**

(22) Date of filing: **03.10.80**

(60) Publication number of the earlier application in accordance with Art. 76 EPC: **0 026 932**

(54) **Stable aqueous emulsions of vinyl acetate polymers, process of their production and surfactant used in said emulsions.**

(30) Priority: **03.10.79 US 81367**
**03.10.79 US 81322**

(43) Date of publication of application:
**03.04.85 Bulletin 85/14**

(45) Publication of the grant of the patent:
**07.10.87 Bulletin 87/41**

(84) Designated Contracting States:
**BE DE FR GB IT NL**

(56) References cited:
**EP-A-0 005 221**
**BE-A- 630 043**
**US-A-4 148 746**

**CHEMICAL ABSTRACTS, vol. 74, no. 16, 19th April 1971, page 61, no. 77492x, Columbus, Ohio, USA; & JP - A - 70 36914 (MITSUBISHI RAYON CO., LTD.) 24-11-1970**

**The file contains technical information submitted after the application was filed and not included in this specification**

(73) Proprietor: **GAF CORPORATION**
**1361 Alps Road**
**Wayne, NJ 07470 (US)**

(72) Inventor: **Chakrabarti, Paritosh M.**
**1361 Redfern Drive**
**Pittsburgh Pennsylvania 15241 (US)**
Inventor: **Kirchner, Darrell G.**
**SR Box 18-12**
**Glenwood New Jersey 07418 (US)**

(74) Representative: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 135 661 B1

Courier Press, Leamington Spa, England.

**Description**

The present patent is based on a divisional application, which has been divided out of the application 80 106 015.3, having the publication No. 0 026 932.

Surfactants sold under the trademark Triton® by Rohm & Haas Co. and Alipal® by GAF Corp. are generally alkyl aryl polyether alcohols and sulfate salts thereof with the exception of Alipal SE 463 and the Triton X 200 series, which are sulfonated components, however, containing 3 or at the most 4 ethylene oxide units in the polyether chain. These sulfonates offer may diversified surfactant properties not commonly encountered in a single compound. They out perform comparable sulfates in offering resistance to decomposition under both highly acidic and basic conditions, thus being suitable in metal cleaning applications for either acid pickling baths or alkaline cleaning formulations. Their excellent compatibility with alkaline detergent builders and their excellent emulsifying character with fats, greases, oils and gelatins afford many application possibilities in lime soap formulation, the film coating industry, as dye leveling agents, post-latex stabilizers and in emulsion polymerization. A major application for this type of surfactant is a shampoo base for the cosmetics industry where it finds wide applicability because of its detergent, latherin and solubilizing properties.

It has been found that in certain applications, as for example the production of high solids content, polyvinylacetate emulsions, coagulation occurs when attempting to produce emulsions having less than 40% water.

The production of high solids content latexes has long been considered to be desirable, the advantage being the maximizing use of production equipment and the minimization of the quantity of water which must be shipped in a latex. High solids content emulsions tend to coagulate immediately or become unstable after relatively short periods of storage. For example, in the production of vinyl acetate, the preparation of high solids content emulsions has necessitated the use of phosphate type surfactants, protective colloids and/or special processing as disclosed for example in US—A—3 637 563. The heretofore employed procedures and formulations has proven to be inadequate because of unfavorable economic factors and/or environmental considerations. Although it would be highly desirable to eliminate the use of a protective colloid thereby providing a cost reduction in the system, repeated attempts to produce such systems have proven unsuccessful. US—A—3 637 563 which is directed to the production of high solid aqueous polymer emulsions suggests the use of surfactants such as, alkylphenoxy poly (ethyleneoxy) ethanols, as well as use of higher molecular weight sulfates and sulfonates. Amount the materials specifically referred to in the aforenoted patent are alkylphenoxy poly (ethyleneoxy) ethanols which contain from about 30 to about 100 ethyleneoxy units, and typical anionic surfactants, e.g., an ethoxylated higher fatty acid which has also been sulfonated. In order to produce a stable emulsion employing the disclosure of 3,637,563, it is necessary to employ one or more protective colloids, particularly when a reflux type polymerization is carried out by using vinyl acetate, or the like, as part of the monomer charge. Included among such materials are either linkage containing protective colloids, such as hydroxy methyl cellulose, hydroxy ethyl cellulose, ethyl hydroxy ethyl cellulose, carboxy methyl cellulose and ethoxylated starch derivatives. Other protective colloid forming substances, i.e., those containing no ether linkages, are also disclosed as being usable, either alone or together with the aforementioned ether linkage-containing materials.

It has now been found that alkylarylpoly(ethyleneoxy)sulfonates of the general formula:

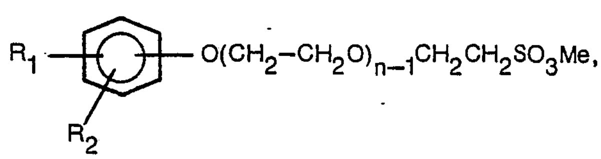

wherein:

$R_1$ is an alkyl group having from 6 to 18 carbons;

$R_2$ is H or an alkyl group having from 6 to 12 carbons;

n is greater than 9; and

Me is a monovalent cation selected from the group consisting of $NH_4$, Na, Li and K,

are useful surfactants for the production of stable polyvinylacetate emulsions.

A further object of the invention is therefore directed to stable aqueous emulsions of a vinyl acetate polymer produced by polymerizing a monomer charge comprising:

a. water;

b. vinyl acetate monomer;

c. at least a second copolymerizable monomer; and

d. at least 0.1 percent by weight based on the weight of the monomers, of a surfactant having the formula:

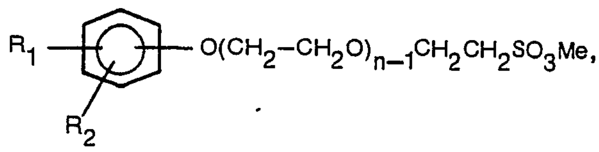

wherein:

$R_1$, $R_2$, n and Me are defined as above;

said water comprising less than 60 percent by weight of the emulsion.

A still further object of the invention is a method of producing aqueous polymer emulsions characterized that a mixture of water, vinyl acetate monomer, at least a second copolymerizable monomer and at least 0.1 percent by weight based on the total weight of the said monomers of a surfactant is contacted and polymerized, the surfactant having the formula

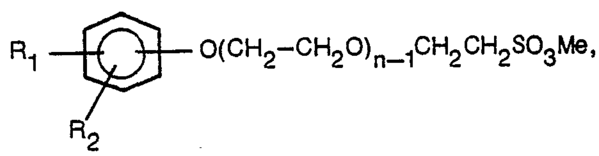

wherein:

$R_1$ is an alkyl group having from 6 to 18 carbon atoms;

$R_2$ is H or an alkkyl group having from 6 to 12 carbon atoms;

n is greater than 9; and

Me is a monovalent cation selected from the group consisting of $NH_4$, Na, Li and K, and

the weight ratio of water to vinyl acetate being predetermined such that the resultant emulsion contains at least 40 percent by weight of solids.

Preferably the combined amount of carbon atoms in $R_1$ and $R_2$ is not greater than 24 carbon atoms, n is preferably from 15 to 30. In a preferred embodiment the surfactant is the sodium salt of nonyl phenoxy poly (ethyleneoxy) sulfonate and contains about 20 ethylene oxide units.

The vinyl acetate latexes according to the invention have high shelf-lives and can be produced without the need to resort to special processing or protective colloids.

In order that those skilled in the art can more fully understand the present invention, the following detailed description and examples are provided. These examples are intended solely for the purpose of illustrating the invention, and are not to be construed as expressing limitations unless so set forth in the appended claims. All parts and percentages are by weight, unless otherwise stated.

Salts of non-ionic surfactants of the type sold under the trademark ALIPAL® SE—463, by GAF Corporation, are generally recognized as having utility in emulsion polymerization. These surfactants are represented by the formula:

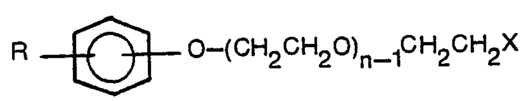

wherein R is an alkyl residue, n = 3 and X = $-SO_3Na$.

Igepal® is a Trade Mark of GAF Corp. wherein X in the above form is —OH. In Igepal CO—630, R is nonyl phenyl and n is 9; in Igepal CO—850, R is nonyl phenyl and n is 20; and in Igepal CO—890, R is nonyl phenyl and n is 40.

Table I compares the use of various surfactants in the emulsion polymerization of vinyl acetate, surfactants having 9 or less repeating ethyleneoxy groups were found to be unable to give the results attainable with longer polymer chains.

Basically, the technology of producing the sulfonates involves initially transforming the terminal alcohol functionality of a nonionic surfactant to a terminal chloride using thionyl chloride, or other chlorinating agent as follows:

1) $$R'O(CH_2CH_2O)_nCH_2CH_2OH \xrightarrow{SOCl_2} R'O(CH_2CH_2O)_nCH_2CH_2Cl$$

wherein $R'=R—C_6H_4$, R being defined as above.

The chloride terminated material is then converted to the sodium sulfonate, using sodium sulfite, as follows:

2) $$R'O(CH_2CH_2O)_nCH_2CH_2Cl \xrightarrow{Na_2SO_3} R'O(CH_2CH_2O)_nCH_2CH_2SO_3Na$$

Looking now in greater detail to the chemistry of the production of the surfactants of the present invention it is noted that the synthetic approach to the production of poly ether sulfonated type surfactants involves the reaction of chlorine capped nonionics with sodium sulfite, as disclosed in US—A—2,115,192.

$$R'(OC_2H_4)_nCl + Na_2SO_3 \xrightarrow[H_2O]{OH-} R'(OC_2H_4)_nSO_3^-Na^+ + NaCl$$

The sulfonate functonality has much less tendency to hydrolize in solutions of low pH than dos an analagous surfactant with a sulfate functionality.

3) $$R''CH_2SO_3Na \xrightarrow{H+} \text{Stable (sulfonate)}$$

4) $$R''CH_2OSO_3Na \xrightarrow{H+} RCH_2OH \text{ (Sulfate decomposition)}$$

R'' being an organic residue.

The ether sulfonates are noted to contain both an ethoxylated nonionic portion and a sulfonic acid anionic portion.

In the systems of Table I, the alkyl aryl poly ethoxy sulfonates were employed for producing poly vinyl acetate latexes, without the incorporation of a protective colloid. In each case, the alkyl sulfonates failed to produce a stable vinyl acetate latex in the absence of a protective colloid.

The criticality of the length of the poly ethoxy group is evident from the failure of the Igepal 630 sulfonate to produce a stable latex. By way of contrast, the sulfonate of Igepal CO—850 formed very stable vinyl acetate emulsions at 55 and 62% solids. The latexes exhibited good mechanical stability, no coagulum, low viscosity and excellent shelf life. The latex produced from the sulfonate of Igepal CO—890 coagulated after 24 hours storage, the probable contributing factor being low conversion to the sulfonate form.

Looking now in greater detail to the chemistry of the production of the surfactants of the present invention it is noted that the synthetic approach to the production of poly ether sulfonate type surfactants involves the reaction of chlorine capped nonionics with sodium sulfite, as disclosed in US—A—2,115,192.

$$R'(OC_2H_4)_nCl + Na_2SO_3 \xrightarrow[H_2O]{OH-} R'(OC_2H_4)_nSO_3^-Na^+ + NaCl$$

The reaction requires high temperature — 155—170°C being the most desirable range — and pressures of about 6 to 9 bar (70—115 psig). To attain over 90% conversion of the chlorine terminated nonionics, reaction times of about 20 hours are typical. The rate of sulfonation appears to be 100 to 500 times the rate of hydrolysis under proper reaction conditions such that good conversions can be expected with most nonionic types under the reaction conditions to be defined in this report. The competing hydrolysis reaction results in a portion of OH terminated nonionics being present in the reaction product as depicted below.

$$R'(OC_2H_4)_nCl \xrightarrow[\Delta]{OH^-/H_2O} R'(OC_2H_4)_nOH \text{ (Hydrolysis)}$$

The sulfonate method of preparation is well-known to those skilled in the art as represented by patents such as US—A—4,091,014, 2,209,911 and 2,148,432.

US—A—4,091,014 is noted to disclose the manufacture of ether sulfonates, by sulfonating alcohols such as:

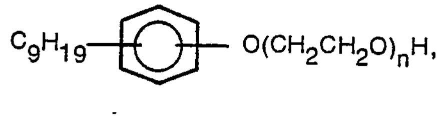

where n is an integer such as 3, 4 and 5. US—A—2 209 911 discloses the production of alkylated phenoxy ether halides, while US—A—2,148,432 discloses the method of producing alkyl phenoxy polyethylene oxide sulfonates from the corresponding alkyl phenoxy poly ethoxy halide. It should thus be evident that the sodium salts of polyether sulfonates of the present invention can be made in acordance with the teachings of the aforenoted patents.

Whereas the prior art typically is primarily concerned with sulfonates having 2, 3 or 4 ethylene oxide units, one aspect of the instant invention is directed to higher values of "n". It appears that the ethylene oxide chain length has an effect on the rate of the sulfonation reaction, based on the unreacted sulfite present in a series of identical runs after a 10 hour reaction period at elevated temperature. It is believed that the steric hinderance of the polyethoxy group is a limiting factor in the sulfonate conversion and precludes adequate conversion of n = 40 polyethyleneoxy to useful sulfonates.

The upper limit for the length of the ethylene oxide chain is not as critical as the lower limit. An upper limit for n, of about 30 is preferred, however, to the extent such a material could be made and isolated, an n value in excess of 40 could be used. It is preferable to balance the properties of the alkyl group and the polyethoxy group. Therefore, the alkyl group should have between 6 and 18 carbons and preferably no more than 12 carbons. While dialkyl groups can be employed, the number of carbons in each alkyl group should not be greater than 12. The conversions are listed below.

Igepal CO Series Conversions at 160°C.

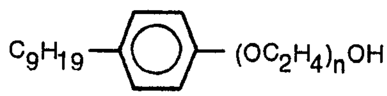

| Name | n | Conversion of Sulfite | Conversion of Sulfite |
|---|---|---|---|
| Igepal CO—630 | 9 | 92 | 71 |
| Igepal CO—730 | 15 | 48 | 37 |
| Igepal CO—850 | 20 | 76 | 34 |

Although a comparison of sulfonate conversion by methylene blue activity may not be accurate through the Igepal CO series, the presence of unreacted sulfite indicates a reduced reactivity of the chlorine terminated nonionic with increasing chain length whatever the product distribution from the chloride might be. Inclusion of a more reactive halogen (NaBr, NaI) can be employed to improve the nonionic reactivity by in situ halogen exchange.

$$R'(OC_2H_4)_nCl \xrightarrow[-NaCl]{NaBr} R'(OC_2H_4)_nBr \xrightarrow{Na_2SO_3} R'(OC_2H_4)_nSO_3Na + NaBr$$

Alternatively, the charge of $Na_2SO_3$ can be increased to increase the reactive collison frequency. In the case of Igepal CO—850 at 50% exc ess of sulfite would result in an increase in salt content in the product of only 2.0%, thus increasing the salt content in the final product but to a tolerable level.

1. Preparation of Nonylphenol + 9 EO Ether Sulfonate

a. *Chlorination*

A 3 l. flask equipped with mechanical stirrer, thermometer, dropping funnel and condenser adapted to a caustic scrubber was charged with 2000 g of Igepal CO—630 (3.28 moles). To it were added 540 g of thionyl chloride (4.54 moles) maintaining the reaction temperature below 60°C. When the addition was completed the reaction mixture was heated to 120°C for ½ hour at which time an IR revealed no OH band at 3500 $cm^{-1}$. The condenser was removed and heated at 100—110°C continued with removal of $SO_2$ by a subsurface nitrogen purge. The heating was continued for a total of 7 hours whereupon conversion to the chloride was essentially complete. The appearance of the chloride was improved by bleaching with 50 g of 30% $H_2O_2$ at 70—90°C for 15 minutes (VCS 18 initial, VCS 13 final). Analysis: Cl 5.51/5.63 (actual/theory).

b. *Sulfonation*

A charge of 638 g of the Igepal CO—630 chloride (1.0 mole) was combined with 131 g of $Na_2SO_3$ (1.0 mole), 1200 ml of distilled water and 6.0 g of 50% NaOH in a 1 gallon (3.785 liter) autoclave and reacted at 160—5°C for 20 hours at 1750 rpm. The product was cooled to 40° and discharged. It was a very viscous clear yellow liquid having a M.B. activity of 25.5% (71.4%) based on a molecular weight of 698, a chloride content of 2.98% (101%) and a VCS of 2. The excess sulfite was removed by the addition of 16 gms of 30% $H_2O_2$ and the pH adjusted to 7.4 with 9 gms of 50% NaOH. The final product properties include 1.48% $Na_2SO_4$, and 40.3% total solids.

2. Preparation of Nonylphenol and 20 EO Ether Sulfonate

The procedure employed for producing the nonylphenol 9 EO Ether sulfonate can be followed using

Igepal CO—850 and thionyl chloride in a 1 to 1.5 mole ratio in the chlorination procedure and a 1 to 1 Igepal CO—850 chloride to sodium sulfite mole ratio in the sulfonation procedure.

3. Preparation of Nonylphenol and 40 EO Ether Sulfonate

The procedure for producing the nonylphenyl 9 E.O. ether sulfonate can be followed, using Igepal CO—890 in approximately a 1 to 1.5 mole ratio with thionyl chloride. The sulfonation step can employ the resultant chloride of Igepal CO—890 in a 1 to 4 mole ratio with sodium sulfite.

Table I sets forth experimental results of the production of high solids content vinyl acetate emulsions using various surfactants. The term "high solids content" as employed herein means emulsions in which the solids constitutes at least 40% by weight of the composition.

The sodium sulfonate of Igepal CO—630 was found to coagulate during its formulation into a vinyl acetate latex but only during the last stages of addition thus showing a higher level of compatibility with vinyl acetate than many other surfactants, but, nevertheless, failing to produce the required results.

The surfactants Alfol® (Trade Mark of Continental Oil Comp.) and Emulphogene® (Trade Mark of GAF Corp.), the sodium salts of which are used in the following Tables as comparison with the sodium sulfonate salts of the other compounds in Table I, are alkyl (polyethyleneoxy) sulfonates with the stated amount of ethyleneoxy groups (n).

TABLE I

| Surfactant | Vinyl Acetate Latex % Solids | Coagulum | Mechanical Stability | Brookfield Viscosity | Polyvinyl Acetate Latex % Solids | pH | Surface Tension |
|---|---|---|---|---|---|---|---|
| Igepal CO—630 — Sulfonate*** | 55 | 100%* | — | — | — | — | — |
| Igepal CO—850 — Sulfonate | 55 | None | OK | 70(1—60) | 56.8 | 3.0 | 43.4 |
| Igepal CO—850 — Sulfonate | 55 | 5.0 g | OK | 95(1—60) | 56.6 | 6.9 | 38.5 |
| Igepal CO—850 — Sulfonate | 55 | None | N.G. | 850(3—60) | — | — | 50.0 |
| Igepal CO—850 — Sulfonate | 55 | None | OK | 85(1—60) | 55.6 | 3.0 | 42.0 |
| Igepal CO—850 — Sulfonate | 55 | None | OK | 385(1—12) | 62.8 | 2.9 | 44.0 |
| Igepal CO—890 — Sulfonate | 62 | Very Slight** | OK | 120(1—30) | 56.2 | 3.1 | 47.0 |
| (Alfon 1012 + 3 E.O.) Sulfonate*** | 55 | 100% | — | — | — | — | — |
| (Alfol 12 + E.O.) Sulfonate*** | 55 | 100% | — | — | — | — | — |
| (Alfol 1214 + 4 E.O.) Sulfonate*** | 55 | 100% | — | — | — | — | — |
| Emulphogene BC—720 Sulfonate*** (n > 9) | 55 | 100% | — | — | — | — | — |
| (Oxotridecyl + 4.2 E.O.) Sulfonate*** | 55 | 100% | — | — | — | — | — |

*In final states of addition
**Coagulated after 24 hours storage
***Comparison

The concentration of the surfactant in the system is not narrowly critical and generally, a surfactant concentration range from 0.1 to 10 percent by weight of total monomer concentration gives the desired result.

Analytical Techniques

a. Coagulum — Solids which were held in the 0.025 mm (60 mesh) screen are thoroughly washed, dried in a 110°C oven for two hours and weighed. When the amount of solid present is too small to collect, or if the coagulum is water soluble, a qualitative description is recorded.

b. Mechanical Stability — The filtered latex is placed in an Osterizer blender, and blended for 10 minutes at maximum speed. If the latex coagulates during this time, mechanical stability is no good.

c. Brookfield Viscosity — The filtered latex sample is poured into a ¼ l sample bottle. The viscometer is fitted with the proper spindle, set for the proper rpm, and lowered into the latex. The viscosity is read directly from a dial.

d. % Solids — Three weighing pans are tared, abut 5 g. of the latex is placed in each, and the samples are placed in a 110—120°C oven for 2 hours. The dried sample is then weighed, and % solids is calculated from (dry weight/wet weight) × 100.

e. Surface tension is measured with a Fisher Tensiomat.

f. pH is measured with any available pH meter.

**Claims**

1. The method of producing aqueous polymer emulsions characterized that a mixture of water, vinyl acetate monomer, at least a second copolymerizable monomer and at least 0.1 percent by weight based on the total weight of the said monomers of a surfactant is contacted and polymerized, the surfactant having the formula:

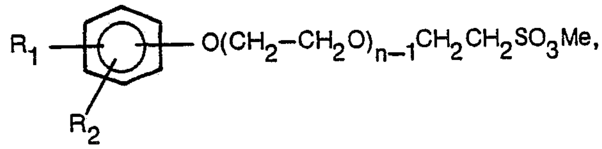

wherein:

$R_1$ is an alkyl group having from 6 to 18 carbon atoms;

$R_2$ is H or an alkyl group having from 6 to 12 carbon atoms;

n is greater than 9; and

Me is a monovalent cation selected from the group consisting of $NH_4$, Na, Li and K, and

the weight ratio of water to vinyl acetate being predetermined such that the resultant emulsion contains at least 40% by weight of solids.

2. The method of claim 1 wherein the combined amount of carbon atoms in $R_1$ and $R_2$ is not greater than 24 carbon atoms.

3. The method of claim 1 or 2, wherein n is from 15 to 30.

4. The method of any of the claims 1 to 3, wherein the surfactant is the sodium salt of nonyl phenoxy poly(ethyleneoxy) sulfonate and contains about 20 ethylene oxide units.

5. A stable aqueous emulsion of a vinyl acetate pplymer produced by polymerizing a monomer charge comprising:

a. water;

b. vinyl acetate monomer;

c. at-least a second copolymerizable monomer; and

d. at least 0.1 percent by weight based on the weight of the monomers, of a surfactant having the formula:

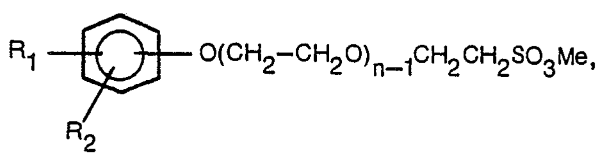

wherein:

$R_1$, $R_2$, n and Me are defined as above;

said water comprising less than 60% by weight of the emulsion.

6. The compound having the formula:

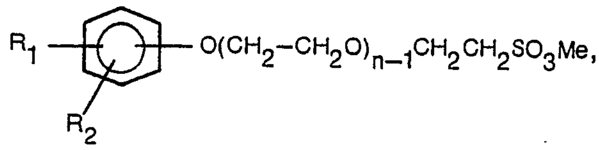

wherein:
$R_1$ is an alkyl group having from 6 to 18 carbons;
$R_2$ is H or an alkyl group having from 6 to 12 carbons;
n is greater than 9; and
Me is a monovalent cation selected from the group consisting of $NH_4$, Na, Li and K.

**Patentansprüche**

1. Ein Verfahren zur Herstellung wässriger Polymeremulsionen, dadurch gekennzeichnet, daß eine Mischung aus Wasser, Vinylacetatmonomer, mindestens einem zweiten, copolymerisierbaren Monomer und mindestens 0,1 Gew.-%, bezogen auf das Gesamtgewicht dieser Monomeren, eines oberflächenaktiven Mittels in Berührung gebracht und polymerisiert wird, wobei das oberflächenaktive Mittel die Formel

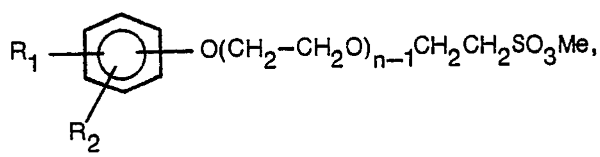

hat, worin
$R_1$ eine Alkylgruppe mit 6 bis 18 Kohlenstoffatomen ist;
$R_2$ H oder eine Alkylgruppe mit 6 bis 12 Kohlenstoffatomen ist;
n = größer als 9 ist; und
Me ein einwertiges Kation, ausgewählt aus der aus $NH_4$, Na, Li und K bestehenden Gruppe, ist, und das Gewichtsverhältnis von Wasser zu Vinylacetat so vorherbestimmt wird, daß die erhaltene Emulsion mindestens 40 Gew.-% Feststoffe enthält.

2. Das Verfahren nach Anspruch 1, worin die kombinierte Menge der Kohlenstoffatome in $R_1$ und $R_2$ nicht größer als 24 Kohlenstoffatome ist.

3. Das Verfahren nach Anspruch 1 oder 2, worin n = von 15 bis 30 ist.

4. Das Verfahren nach irgendeinem der Ansprüche 1 bis 3, worin das oberflächenaktive Mittel das Natriumsalz von Nonylphenoxypoly-(ethylenoxy)-sulfonat ist und etwa 20 Ethylenoxideinheiten enthält.

5. Eine stabile wässrige Emulsion aus einem Vinylacetatpolymer, hergestellt durch Polymerisieren einer Monomerbeschickung, die umfaßt
a. Wasser,
b. Vinylacetatmonomer,
c. mindestens ein zweites, copolymerisierbares Monomer, und
d. mindestens 0,1 Gew.-%, bezogen auf das Gewicht der Monomeren, eines oberflächenaktiven Mittels mit der Formel

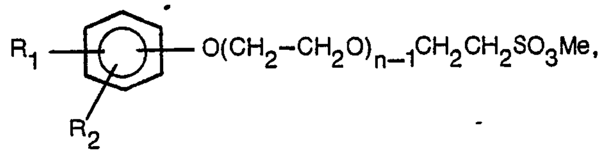

worin
$R_1$, $R_2$, n und Me wie oben definiert sind, wobei das Wasser weniger als 60 Gew.-% der Emulsion ausmacht.

6. Eine Verbindung mit der Formel

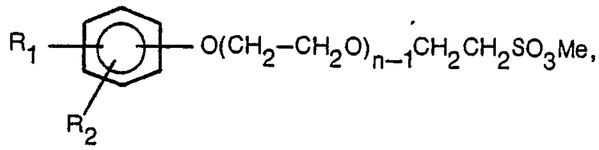

worin
$R_1$ eine Alkylgruppe mit 6 bis 18 Kohlenstoffatomen ist,
$R_2$ H oder eine Alkylgruppe mit 6 bis 12 Kohlenstoffatomen ist,
n = größer als 9 ist, und
Me ein einwertiges Kation, ausgewählt aus der aus $NH_4$, Na, Li und K bestehenden Gruppe, ist.

**Revendications**

1. Procédé de production d'émulsions aqueuses de polymères, caractérisé en ce que les composants d'un mélange d'eau, d'acétate de vinyle monomère, d'au moins un second monomère copolymérisable et d'au moins 0,1% en poids, sur la base du poids total desdits monomères d'un surfactant sont mis en contact et polymérisés, le surfactant répondant à la formule:

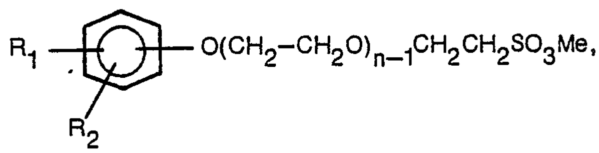

dans laquelle:
$R_1$ est un groupe alkyle ayant 6 à 18 atomes de carbone;
$R_2$ représente H ou un groupe alkyle ayant 6 à 12 atomes de carbone;
*n* est supérieur à 9; et
Me est un cation monovalent choisi dans le groupe comprenant $NH_4$, Na, Li et K, et
le rapport en poids de l'eau à l'acétate de vinyle est prédéterminé de manière que l'émulsion résultante contienne au moins 40% en poids de matières solides.

2. Procédé suivant la revendication 1, dans lequel le quantité totale d'atomes de carbone dans $R_1$ et $R_2$ ne dépasse pas 24 atomes de carbone.

3. Procédé suivant la revendication 1 ou 2, dans lequel *n* a une valeur de 15 à 30.

4. Procédé suivant l'une quelconque des revendications 1 à 3, dans lequel le surfactant est le sel de sodium d'un nonylphénoxypoly(éthylèneoxy)sulfonate et contient environ 20 motifs oxyde d'éthylène.

5. Emulsion aqueuse stable d'un polymère d'acétate de vinyle, produite par polymérisation d'une charge de monomères comprenant:
a. de l'eau;
b. de l'acétate de vinyle monomère;
c. au moins un second monomère copolymérisable; et
d. au moins 0,1% en poids, sur la base du poids des monomères, d'un surfactant répondant à la formule:

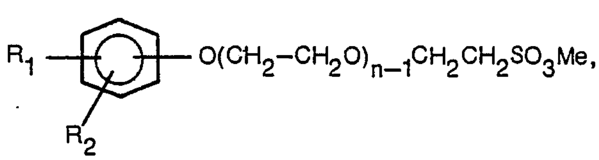

dans laquelle:
$R_1$, $R_2$, *n* et Me sont tels que définis ci-dessus;
l'eau constituant moins de 60% en poids de l'émulsion.

6. Composé de formule:

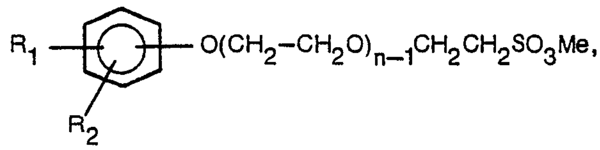

dans laquelle:

$R_1$ est un groupe alkyle ayant 6 à 18 atomes de carbone;

$R_2$ représente H ou un groupe alkyle ayant 6 à 12 atomes de carbone;

*n* est supérieur à 9; et

Me est un cation monovalent choisi dans le groupe comprenant $NH_4$, Na, Li et K.